(19) Europäisches Patentamt
European Patent Office
Office européen des brevets

(11) Publication number: 0 379 059
A1

# (12) EUROPEAN PATENT APPLICATION

(21) Application number: 90100494.5

(22) Date of filing: 11.01.90

(51) Int. Cl.5: C07C 251/48, C07C 235/56, C07C 235/64

(30) Priority: 17.01.89 US 297894

(43) Date of publication of application:
25.07.90 Bulletin 90/30

(84) Designated Contracting States:
AT BE CH DE ES FR GB IT LI NL SE

(71) Applicant: THE DOW CHEMICAL COMPANY
2030 Dow Center Abbott Road
Midland, MI 48640(US)

(72) Inventor: Puckett, Paul M.
126 Daisy
Lake Jackson Texas 77566(US)
Inventor: Earls, Jimmy D.
209 Banyan
Lake Jackson Texas 77566(US)
Inventor: Cavitt, Michael B.
303 South Yaupon
Lake Jackson Texas 77566(US)

(74) Representative: Sternagel, Hans-Günther, Dr. et al
Patentanwälte Dr. Michael Hann Dr. H.-G.
Sternagel Sander Aue 30
D-5060 Bergisch Gladbach 2(DE)

## (54) Dihydroxybenzophenone oximes, their preparation and rearrangement to the anilide.

(57) Dihydroxybenzophenone oximes are prepared by reacting a dihydroxybenzophenone with an acid adduct of hydroxylamine in the presence of a base and an inert solvent or reaction medium and dihydroxybenzanilides are prepared by the thermal rearrangement of dihydroxybenzophenone oximes. The rearrangement reaction can optionally employ acid catalysts for conversion of the oximes at lower temperatures.

EP 0 379 059 A1

## DIHYDROXYBENZOPHENONE OXIMES, THEIR PREPARATION AND REARRANGEMENT TO THE ANILIDE

The present invention pertains to dihydroxybenzophenone oximes and isomers and analogs thereof, a method for their preparation and their rearrangement to 4,4'-dihydroxybenzanilide.

Makaruk, Polanska and Wazynska in "PREPARATION AND PROPERTIES OF THERMOTROPIC POLYAMIDOESTERS", Am. Chem. Soc., Div. Polym. Chem., Vol. 24 (2), pp. 260-261 (1983) stated that they prepared 4,4'-dihydroxybenzophenone oxime following the procedure of Bender, Buczkowski and Plenkiewicz in "NMR Study of Certain Aliphatic Azomethines. II. Ketoxime O-Methyl Ethers and Their Complexes with Trimethylaluminum", Bull. Acad. Pol. Sci., Ser. Sci. Chim., Vol. XVII, No. 11-12, pp. 643-647 (1969) by reacting 4,4'-dihydroxybenzophenone with hydroxylamine hydrochloride. Bender et al. reacted the ketone with the $H_2NOCH_3$•HCl instead of $H_2NOH$•HCl. Since Makaruk et al. stated that their 4,4'-dihydroxybenzophenone oxime product was light beige in color and had a melting point of 270° C we do not believe that they actually prepared the oxime of 4,4'-dihydroxybenzophenone since we have observed a white crystalline solid with a melting point in the range of 155° C to 158° C. What Bender et al. actually produced was the anilide derivative which we have observed to be the product which forms by rearrangement upon heating 4,4'-dihydroxybenzophenone oxime.

One aspect of the present invention pertains to dihydroxybenzophenone oximes represented by the following formula I:

Formula I

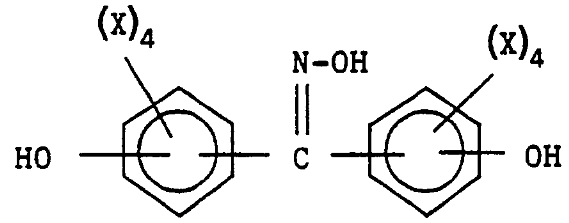

wherein each X is independently hydrogen, a hydrocarbyl or hydrocarbyloxy group having from 1 to 12 carbon atoms, a halogen atom or a cyano group.

Another aspect of the present invention pertains to dihydroxybenzanilides represented by the following formula II:

Formula II

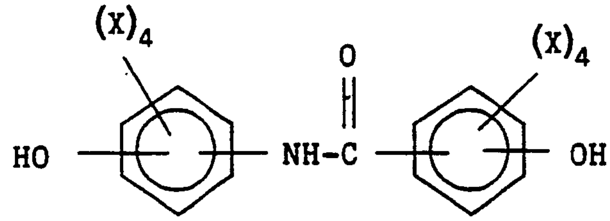

wherein each X is independently hydrogen, a hydrocarbyl or hydrocarbyloxy group having from 1 to 12 carbon atoms, a halogen atom or a cyano group and with the proviso that when both OH groups are in the para position with respect to the amide (-NH-CO-) group, at least one X group is not a hydrogen atom.

Another aspect of the present invention pertains to a process for the preparation of dihydroxybenzophenone oximes by reacting a benzophenone with an acid adduct of hydroxylamine in the presence of a base and a solvent or reaction medium.

Another aspect of the present invention pertains to a process for the preparation of dihydroxybenzanilides by the thermal rearrangement of dihydroxybenzophenone oximes with or without a solvent or reaction medium and optionally in the presence of an acid catalyst.

The dihydroxybenzophenone oximes of the present invention are prepared by reacting the corresponding dihydroxybenzophenone with an acid adduct of hydroxylamine in the presence of a base and a solvent or reaction medium at a temperature suitably from 0° C to 150° C, more suitably from 20° C to 120° C,

most suitably from 30° C to 100° C; at a pressure suitably from atmospheric to 200 psig, more suitably from zero psig to 100 psig, most suitably from zero psig to 45 psig; for a time suitably from 0.5 to 48, more suitably from 0.5 to 24, most suitably from 1 to 12 hours. The higher reaction temperatures require less reaction time; whereas the lower reaction temperatures require longer reaction times. At temperatures above 150° C, the oxime formed is somewhat unstable and begins to undergo rearrangement. At temperatures below 0° C, the reaction becomes so slow as to be impractical.

Suitable hydroxybenzophenone compounds which can be employed herein include, for example, those represented by the following formula:

Formula I

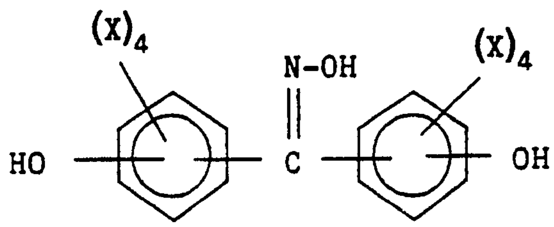

wherein each X is independently hydrogen, a hydrocarbyl or hydrocarbyloxy group having suitably from 1 to 12, more suitably from 1 to 6, most suitably from 1 to 4 carbon atoms, a cyano group, or a halogen atom, suitably chlorine or bromine.

The term "hydrocarbyl" as employed herein means any aliphatic, cycloaliphatic, aromatic, aryl substituted aliphatic or cycloaliphatic, or aliphatic or cycloaliphatic substituted aromatic groups. The aliphatic groups can be saturated or unsaturated. Likewise, the term "hydrocarbyloxy" means a hydrocarbyl group having an oxygen linkage between it and the object to which it is attached.

Acids which form suitable adducts with the hydroxylamine include, for example, any mineral or organic acid, such as HCl, $H_2SO_4$, $H_3PO_4$, formic acid, oxalic acid, acetic acid, propionic acid and combinations thereof.

The hydroxylamine•acid adduct is employed in quantities suitably from 1 to 2, more suitably from 1 to 1.5, most suitably from 1 to 1.2 moles per mole of dihydroxybenzophenone.

Suitable bases which can be employed in the reaction of the dihydroxybenzophenone and hydroxylamine acid adduct include, for example, alkali and alkaline earth metal hydroxides, bicarbonates, carbonates, bisulfates, carboxylates, cyclic amines, aromatic amine, nitrogen-containing heterocycles and combinations thereof. Particularly suitable bases which can be employed herein include, for example, sodium hydroxide, potassium hydroxide, sodium bicarbonate, sodium carbonate, potassium carbonate, calcium carbonate, barium carbonate, sodium bisulfate, potassium acetate, sodium acetate, pyridine, lutidine and combinations thereof.

The base compounds are employed in an amount sufficient to adjust the pH of the reaction mixture to about 4 to 5. These amounts are usually from 1 to 3 equivalents of base per mole of dihydroxybenzophenone.

Suitable solvents or reaction media in which the dihydroxybenzophenone and hydroxylamine•acid adduct can be reacted include, for example, water, alcohols, glycol ethers, cyclic ethers, organic acids, amides, glymes, glycols, aromatic hydrocarbons, aliphatic hydrocarbons, esters and combinations thereof. Particularly suitable solvents or reaction media which can be employed herein include, for example, water, methanol, ethanol, isopropanol, butanol, tetrahydrofuran, dimethylformamide, dioxane, sulfolane, dimethylsulfoxide, ethylene glycol, 2-methoxy-1-ethanol, 1-methoxy-2-propanol, 1,2-dimethoxy ethane, 2-ethoxy-1-ethanol, acetic acid, propionic acid, formic acid, toluene, xylene, decalin and combinations thereof.

The solvents or reaction media are employed in amounts suitably from 10 to 1000, more suitably from 10 to 750, most suitably from 100 to 500 percent by weight based upon the weight of the dihydroxybenzophenone compound.

The rearrangement of the dihydroxybenzophenone oxime to the dihydroxybenzanilide can be conducted at temperatures suitably from 0° C to 200° C, more suitably from 30° C to 160° C, most suitably from 50° C to 150° C; at a pressure suitably from atmospheric to 200 psig, more suitably from zero to 100, most suitably from zero to 45 psig for a time suitably from 0.5 to 48, more suitably from 0.5 to 24, most

suitably from 1 to 12 hours. The higher reaction temperatures require less reaction time; whereas the lower reaction temperatures require longer reaction times. At temperatures above 160° C, the rearrangement occurs very rapidly which can result in a sudden increase to higher temperatures. At temperatures below 0° C, the reaction becomes so slow as to be impractical.

The reaction is preferably conducted in an inert atmosphere which can be provided by a gas such as, for example, nitrogen, helium, neon, argon, krypton, xenon, radon, methane, ethane, propane and combinations thereof. Any means which will exclude the presence of oxygen from the reaction is suitable and preferred.

The rearrangement of the dihydroxybenzophenone oxime can be conducted, if desired, in the presence of an acid catalyst. Suitable such acid catalysts which can be employed herein include, for example, mineral acids, organic acids, or Lewis acids such as toluenesulfonic acid, benzenesulfonic acid, trifluoroacetic acid, sulfuric acid, hydrochloric acid, phosphoric acid, polyphosphoric acid, trichloroacetic acid, $HClO_4$, formic acid, acetic acid, propionic acid, oxalic acid, trichloroacetic acid, chloroacetic acid, carbonic acid, $BF_3$, $AlX_3$, $FeX_3$, $ZnX_2$, $MgX_2$, $Al_2O_3$, (where X is a halogen, particularly Cl, Br or I or OAc), or combinations of such acids. Also suitable acid catalysts which can be employed herein include, for example, acid halides or partial acid halides and acid anhydrides of various mineral acids and organic acids, such as toluenesulfonyl chloride, trifluoroacetic anhydride, acetic anhydride, polyphosphoric acid, phosphoryl chloride, phosphorous pentachloride, phosphorous tribromide, phosphorous pentoxide, trichoroacetyl chloride, phosgene, thionyl chloride, sulfuryl chloride, benzenesulfonyl chloride, acetyl chloride, propionic anhydride, propionyl chloride and combinations thereof.

The acid catalyst is employed in amounts suitably from zero to 100, more suitably from 0.01 to 1, most suitably from 0.01 to 0.5 equivalents per mole of dihydroxybenzophenone oxime compound.

If desired, the rearrangement can be conducted in the presence of a suitable solvent or reaction medium such as organic acids, water, alcohols, cyclic ethers, glycol ethers, glycols, glymes, amides, halogenated hydrocarbons, esters, aliphatic hydrocarbons, aromatic hydrocarbons and combinations thereof. Particularly suitable such solvents or reaction media which can be employed herein include, for example, acetic acid, tetrahydrofuran, methanol, ethanol, propanol, isopropanol, butanol, dimethylformamide, dioxane, trichloromethane, dichloromethane, toluene, xylene, N-methylpyrollidinone, sulfolane, dimethyl sulfoxide, ethylene glycol, 2-methoxy-1-ethanol, 2-ethoxy-1-ethanol, 1,2-dimethoxyethane, 1,2-diethoxyethane, 1-methoxy-2-propanol, decalin, ethyl acetate, water and combinations thereof.

These solvents or reaction media are suitably employed in amounts of from zero to 1000, more suitably from 10 to 800, most suitably from 100 to 500 percent by weight based upon the weight of the dihydroxybenzophenone oxime compound.

The dihydroxybenzanilide compounds can be employed to prepare epoxy resins by reaction with an epihalohdyrin and subsequent dehydrohalogenation in the presence of a basic-acting compound such as alkali metal and alkaline earth metal hydroxides, carbonates and bicarbonates in the manner known to those skilled in the art of preparing epoxy resins.

The following examples are illustrative of the present invention.

Example 1 Preparation of 4,4′-Dihydroxybenzophenone Oxime from 4,4′-Dihydroxybenzophenone

One hundred grams of 4,4′-dihydroxybenzophenone (0.467 mole) was added to 300 ml of ethanol in a stirred 1-liter Erlenmeyer flask. After the 4,4′-dihydroxybenzophenone had dissolved, a solution consisting of 48.6 grams of hydroxylamine hydrochloride (0.699 mole) and 57.4 grams of sodium acetate (0.700 mole) in 70 ml of deionized water was added followed by an additional 100 ml of ethanol. This mixture was stirred and heated on a hot plate to a gentle refluxing condition (75° C). After heating for 4 hours, the solution was allowed to cool to room temperature with stirring and then filtered. One hundred (100) ml of ethanol was used to wash the filter cake. The total filtrant obtained (600.4 grams) was then concentrated to a weight of 219.2 grams by evaporation of the ethanol. This solution was then placed in a stirred 1-liter Erlenmeyer flask to which 600 ml of deionized water was added. With the addition of the deionized water, a white precipitate was formed. After 30 minutes of stirring, this solution was filtered. The solids obtained weighed 98.22 grams after drying (yield = 91.8 percent). Fourier transform infrared (FTIR) analysis of this material showed absorbances at the following wave numbers which are indicative of the structure for 4,4′-dihydroxybenzophenone oxime (Formula III): 3400 $cm^{-1}$ (-OH stretch), 1607 $cm^{-1}$ and 1513 $cm^{-1}$ (C-C stretch/aromatic ring) and 1235 $cm^{-1}$ (aromatic C-0 stretch). Differential scanning calorimetry (DSC) analysis of this product showed a melt endotherm at 155° C followed by an exotherm (rearrangement of 4,4′-dihydroxybenzophenone oxime to 4,4′-dihydroxy benzanilide) at 155-180° C ($\Delta H$ = -580 joules/gram).

Following this exotherm a melt endotherm for the rearrangement product was observed at 269° C. Analysis of the 4,4′-dihydroxybenzophenone oxime product by liquid chromatography indicates 97.8 percent purity.

Formula III

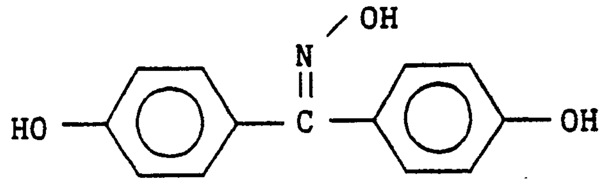

4,4'-Dihydroxybenzophenone Oxime

Example 2 Preparation of 4,4′-Dihydroxybenzanilide from 4,4′-Dihydroxybenzophenone oxime

Sixty-six grams of the 4,4′-dihydroxybenzophenone oxime (0.288 mole) obtained from Example 1 was added to 330 ml of glacial acetic acid in a 500 ml round bottom flask equipped with a stirrer, water cooled condenser, nitrogen purge, and heating mantle. A catalytic amount of toluenesulfonic acid (1.85 grams, 0.027 mole) was next added and the reaction mixture was then heated to 83° C. After heating for approximately 2 hours, a precipitate began to form. The reaction mixture was stirred for an additional 2 hours at 87° C. Twenty-five (25) ml of deionized water was next added and after 30 minutes, the contents of the reaction flask were transferred to a stirred, 1-liter Erlenmeyer flask. Immediately following this transfer, 400 ml of deionized water was added. This solution was stirred for 45 minutes and then filtered. The filter cake obtained was washed with 800 ml deionized water and then dried. The resultant solids, which were a light beige color, weighed 54.82 grams (yield to 4,4′-dihydroxybenzanilide from oxime conversion = 83.1 percent). FTIR analysis of this product showed absorbances at the following wave numbers which are indicative of the structure for 4,4′-dihydroxybenzanilide (Formula IV): 3322 $cm^{-1}$ (N-H stretch), 1646 $cm^{-1}$ (Amide I band), 1609 $cm^{-1}$ and 1514 $cm^{-1}$ (C-C stretch/aromatic ring), 1540 $cm^{-1}$ (Amide II band), and 1251 $cm^{-1}$ (aromatic C-0 stretch). DSC analysis showed a sharp melt endotherm at 273° C.

Formula IV

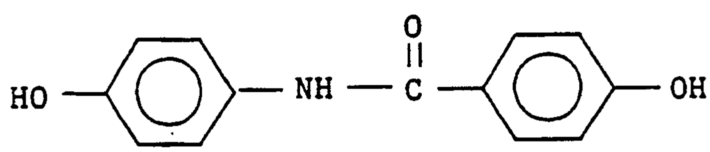

4,4'-Dihydroxybenzanilide

Example 3 Preparation of 4,4′-Dihydroxybenzanilide oxime from 4,4′-Dihydroxybenzophenone

One hundred grams of 4,4′-dihydroxybenzophenone (0.467 mole), 1000 grams of 2B alcohol, 750 grams of water and 34.7 grams of hydroxylamine hydrochloride (0.499 moles) were added to a 3 liter flask. This reaction mixture was stirred until the 4,4′-dihydroxybenzophenone and hydroxylamine hydrochloride were dissolved, then 375 grams of an aqueous solution containing 25 weight percent KOH (1.67 moles) was added dropwise over a 6 hour period. This mixture was then refluxed for 48 hours after which it was cooled and filtered. The pH of the filtrate was adjusted to approximately 2 with concentrated HCl and then evaporated down in vacuo. The solids obtained were recrystallized from 2B alcohol. The oxime product recovered from this recrystallization weighed 97.2 grams--after drying (yield = 90.8 percent). NMR analysis of this material is indicative of the structure for the oxime.

Example 4 Preparation of 4,4′-Dihydroxybenzanilide from 4,4′-Dihydroxybenzophenone Oxime

Part of the 4,4′-dihydroxybenzophenone oxime (4.7 grams) obtained from Example 3 was heated at 150° C -160° C for 1.5 hours. The dark residue obtained after heating was recrystallized twice from an ethanol-water mixture. The 4,4′-dihydroxybenzanilide product recovered from this recrystallization weighed 3.0 grams after drying (yield = 63.8 percent). The melting point of this material was 265° C - 267° C and NMR analysis is indicative of the structure for 4,4′-dihydroxybenzanilide.

Example 5 Epoxidation of 4,4′-Dihydroxybenzanilide

4,4′-dihydroxybenzanilide (99.6 grams, 0.434 moles) prepared from the method given in Example 2, epichlorohydrin (804.57 grams, 8.70 moles), deionized water (69.96 grams, 8.0 percent by weight of the epichlorohydrin used) and isopropanol (433.23 grams, 35 percent by weight of the epichlorohydrin used) were added to a round bottom flask and heated to 65° C with stirring under a nitrogen atmosphere. After the temperature had reached 65° C, sodium hydroxide (31.31 grams, 0.78 moles) dissolved in deionized water (125.25 grams) was added dropwise over a one hour period so as to maintain the reaction temperature at 65° C. Fifteen minutes after completion of the aqueous sodium hydroxide addition, the stirring was stopped and the aqueous layer which separates from the reaction mixture was removed and discarded. Stirring was then resumed and a second solution of sodium hydroxide (13.92 grams, 0.35 moles) dissolved in deionized water (55.66 grams) was added over a 30 minute period so as to maintain the reaction temperature at 65° C. Fifteen minutes after completion of the second aqueous sodium hydroxide addition, stirring and heating were stopped and the reaction mixture was transferred to a separatory funnel. The aqueous layer which separates from the reaction mixture was removed and discarded. As the remaining organic layer cools to room temperature, sufficient methylene chloride was added to keep the epoxy resin dissolved in solution. The cooled organic layer obtained was then washed four times with deionized water. The volume of deionized water used during each wash was approximately one half that of the organic layer. The washed organic layer was then rotary evaporated under vacuum at 125° C. The final product obtained after drying was a beige crystalline solid (142.03 grams, yield = 95.8 percent) which had a melting point of 179° C - 186° C and an epoxide equivalent weight of 178.0.

Example 6 Preparation of a Cured Film from the Diglycidyl Ether of 4,4′-Dihydroxybenzanilide

Part of the epoxy resin derivative of 4,4′-dihydroxybenzanilide prepared from Example 5 (4.5063 grams) was combined and mixed with an equivalent amount of a diamine hardener, sulfanilamide (1.0904 grams). This blend of solids was then placed in a convection oven heated to 175° C. After melting had occurred (approximately 10 minutes), the resinous mixture was poured into a preheated 7.5 inch x 0.5 inch x 0.021 inch (190.5 mm x 12.7 mm x 0.53 mm) mold. This mold was then placed in a mechanical press heated to 160° C. Pressure (4000 psig) was applied to the mold within 5 minutes and after 1.5 hours at 160° C, the temperature was raised to 180° C. After one hour at 180° C, the temperature was raised to 200° C where it was held for 2 hours and then the press was allowed to cool to room temperature. Following cool down, a flexible film was obtained from the mold. The glass transition temperature measured for this polymer by differential scanning calorimetry was 223° C. This value was 35° C higher than that obtained for a diglycidyl ether of Bisphenol A epoxy resin (epoxide equivalent weight = 179.5) which was cured using the same hardener and 4500 PPM tetrabutylphosphonium acetate•acetic acid complex catalyst.

## Claims

1. A dihydroxybenzophenone oxime represented by the following formula I:

Formula I

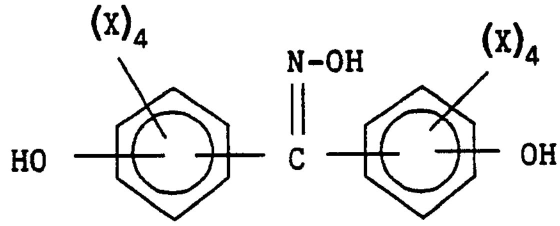

wherein each X is independently hydrogen, a hydrocarbyl or hydrocarbyloxy group having from 1 to 12 carbon atoms, a halogen atom, or a cyano group.

2. A dihydroxybenzophenone oxime of Claim 1 wherein each X is independently hydrogen, a hydrocarbyl or hydrocarbyloxy group having from 1 to 4 carbon atoms, chlorine, bromine, or a cyano group.

3. A 4,4′-dihydroxybenzophenone oxime melting at a temperature below about 170° C.

4. A dihydroxybenzanilide represented by the following formula II:

Formula II

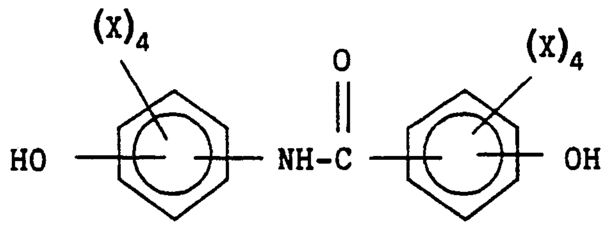

wherein each X is independently hydrogen, a hydrocarbyl or hydrocarbyloxy group having from 1 to 12 carbon atoms, a halogen atom, or a cyano group; with the proviso that when both of the OH groups are in the para position with respect to the amide (-NH-CO-) group, at least one X is not a hydrogen atom.

5. A dihydroxybenzanilide of Claim 4 wherein each X is independently hydrogen, a hydrocarbyl or hydrocarbyloxy group having from 1 to 4 carbon atoms, chlorine, bromine, or a cyano group; with the proviso that when both of the OH groups are in the para position with respect to the amide (-NH-CO-) group, at least one X is not a hydrogen atom.

6. A process for the preparation of dihydroxybenzophenone oximes by reacting a dihydroxybenzophenone with an acid adduct of a hydroxylamine in the presence of a base and a solvent or reaction medium.

7. A process of Claim 6 wherein the reaction is conducted at a temperature of from 0° C to 150° C; said base is an alkali metal or alkaline earth metal hydroxide, carbonate, bicarbonate or carboxylate; and said hydroxylamine is adducted with an organic or mineral acid.

8. A process of Claim 7 wherein the reaction is conducted at a temperature of from 20° C to 120° C; said base is an alkali metal carboxylate; and said solvent or reaction medium is an alcohol.

9. A process of Claim 8 wherein the reaction is conducted at a temperature of from 30° C to 100° C; said base is sodium acetate or potassium hydroxide; and said solvent or reaction medium is ethanol.

10. A process for the preparation of dihydroxybenzanilides by the thermal rearrangement of dihydroxybenzophenone oximes.

11. A process of Claim 10 wherein said thermal rearrangement is conducted at a temperature of from 150° C to 200° C in the absence of an acid catalyst.

12. A process of Claim 11 wherein said thermal rearrangement is conducted at a temperature of from 20° C to 200° C in the presence of an acid catalyst and a solvent or reaction medium.

13. A process of Claim 12 wherein said rearrangement is conducted at a temperature of from 50° C to 150° C; said acid catalyst is toluenesulfonic acid; and said solvent or reaction medium is acetic acid.

European Patent Office

# EUROPEAN SEARCH REPORT

Application Number

EP 90 10 0494

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS vol. 103, no. 11, 16 September 1985, abstract no. 87560z, Columbus, Ohio, USA; I. SHARMA et al.: "Sodium borohydride induced deamination of diarylimines" & Indian J. Chem. Sect. B 1985, vol. 24B, no. 1, pages 59-61<br>--- | 1,2 | C 07 C 251/48<br>C 07 C 235/56<br>C 07 C 235/64 |
| X | CHEMICAL ABSTRACTS vol. 97, no. 8, 23 August 1982, abstract no. 56871e, Columbus, Ohio, USA; & JP - A - 82 49 635 (DAINICHI NIPPON CABLES LTD.) & Formula Index page 1266F, column 2, lines 107,108<br>--- | 1,2 | |
| X | CHEMICAL ABSTRACTS vol. 96, no. 17, 26 April 1982, abstract no. 137272s, Columbus, Ohio, USA; H.M. NORBURY et al.: "The metabolism and disposition of 3,5-dibromosalicylanilide in male and female rats" & Xenobiotica 1981, vol. 11, no. 8, pages 511-518 & Formula index page 994F, column 2, lines 25-28<br>--- -/- | 1,2 | TECHNICAL FIELDS SEARCHED (Int. Cl.5)<br>C 07 C 251/48<br>C 07 C 235/56<br>C 07 C 235/64 |

The present search report has been drawn up for all claims

| Place of search | Date of completion of the search | Examiner |
|---|---|---|
| BERLIN | 06-03-1990 | KAPTEYN H G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 90 10 0494

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS vol. 91, no. 8, 19 November - 3 December 1979, abstract no. 169491a, Columbus, Ohio, USA; H. KAEMMERER et al.: "Preparation of phenolic compounds and phenol/formaldehyde polycondensates with ketoxime, aldoxime, or 1-methyl-4-hydroxyiminomethylpyridinium groups and their effect on parathion-methyl and related compounds" & Makromol. Chem. 1979, vol. 180, no. 7, pages 1635-1650 & Formula index page 1186F, column 2, lines 84,85<br>--- | 1,2 | |
| X | JOURNAL OF ORGANIC CHEMISTRY vol. 48, no. 15, 29 July 1983, pages 2613-2615; G. STOKKER: "Preparation of 1,2-Benzisoxazoles from Salicylaldoximes via Trichloroacetyl Isocyanate" * page 2614, table II *<br>--- | 1,2 | |
| X | CHEMICAL ABSTRACTS vol. 103, no. 8, November 1985, abstract no. 154147k, Columbus, Ohio, USA; & JP - A - 702 114 (UNITED STATES DEPT. OF THE ARMY)<br>--- | 4 | TECHNICAL FIELDS SEARCHED (Int. Cl.5) |
| X | CHEMICAL ABSTRACTS vol. 97, no. 8, July 1982, abstract no. 6006f, Columbus, Ohio, USA; & JP - A - 81 49 905 (MICROBIOCHEMICAL RESEARCH FOUNDATION)<br>--- -/- | 4 | |

The present search report has been drawn up for all claims

| Place of search | Date of completion of the search | Examiner |
|---|---|---|
| BERLIN | 06-03-1990 | KAPTEYN H G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 90 10 0494

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS vol. 72, no. 15, 13 April 1970, abstract no. 78616m, Columbus, Ohio, USA; J.B. BROUGHTON et al.: "Chemotherapy of fascioliasis. III. Amides derived from 4-cyano-2-iodo-6-nitrophenol (nitroxynil) and 2,6-diiodo-4-nitrophenol (disophenol)" & J. Sci. Food Agr. 1970, vol. 21, no. 1, pages 53-56 & Formula Index page 704F, column 2, lines 30-33 --- | 4 | |
| X | CHEMICAL ABSTRACTS vol. 71, no. 19, 10 November 1969, abstract no. 91112t, Columbus, Ohio, USA; M. SCHELLENBAUM et al.: "N-(o-Hydroxyphenyl)salicylamides: bactericides and fungicides" & ZA 68 04 554 (CIBA LTD.) 20.12.1968 --- | 4 | TECHNICAL FIELDS SEARCHED (Int. Cl.5) |
| X | CHEMICAL ABSTRACTS vol. 71, no. 8, August 1969, abstract no. 21823b, Columbus, Ohio, USA; A.W.J. BROOME et al.: "Fasciolicide" & Ind. Chim. Belge 1967, vol. 32 (Spec. No.), page 204-208 --- | 4 | |
| X | JOURNAL OF THE CHEMICAL SOCIETY, DATON TRANSACTIONS 1988, pages 641-645; M. KOIKAWA et al.: "Manganese (IV) and Manganese (V) Complexes with N-(2-Hydroxyphenyl)-salicylamides" * page 641, formulas H3L1,H3L2,H3L3 * ----- | 5 | |

The present search report has been drawn up for all claims

| Place of search | Date of completion of the search | Examiner |
|---|---|---|
| BERLIN | 06-03-1990 | KAPTEYN H G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document